(19) Europäisches Patentamt
European Patent Office
Office européen des brevets

(11) **EP 3 148 411 B1**

(12) **EUROPEAN PATENT SPECIFICATION**

(45) Date of publication and mention
of the grant of the patent:
**22.07.2020 Bulletin 2020/30**

(51) Int Cl.:
*A61B 5/00* *(2006.01)*          *A61B 5/026* *(2006.01)*
*A61B 5/053* *(2006.01)*

(21) Application number: **15729582.5**

(86) International application number:
**PCT/JP2015/002521**

(22) Date of filing: **20.05.2015**

(87) International publication number:
**WO 2015/182065 (03.12.2015 Gazette 2015/48)**

(54) **IMAGING APPARATUS, IMAGING METHOD AND MEDICAL IMAGING SYSTEM**

ABBILDUNGSVORRICHTUNG, ABBILDUNGSVERFAHREN UND MEDIZINISCHES ABBILDUNGSSYSTEM

APPAREIL D'IMAGERIE, PROCÉDÉ D'IMAGERIE ET SYSTÈME D'IMAGERIE MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2014 JP 2014112436**
**24.12.2014 JP 2014260054**

(43) Date of publication of application:
**05.04.2017 Bulletin 2017/14**

(73) Proprietor: **Sony Corporation**
**Tokyo 108-0075 (JP)**

(72) Inventors:
• **NAKAO, Isamu**
**Tokyo 108-0075 (JP)**
• **TANAKA, Eiichi**
**Tokyo 108-0075 (JP)**
• **KISHIMOTO, Takuya**
**Tokyo 108-0075 (JP)**

(74) Representative: **D Young & Co LLP**
**120 Holborn**
**London EC1N 2DY (GB)**

(56) References cited:
**US-B1- 7 113 817**

• **JIANJUN QIU, PENGCHENG LI AND AL: "Spatiotemporal laser speckle contrast analysis for blood flow imaging with maximized speckle contrast", JOURNAL OF BIOMEDICAL OPTICS, vol. 15, no. 1, 15 January 2010 (2010-01-15), XP040513985, DOI: 10.1117/1.3290804**
• **O. B. THOMPSON ET AL: "Measuring Doppler-like power spectra and dermal perfusion using laser speckle contrast with multiple exposures", PROCEEDINGS OF SPIE, vol. 7563, 11 February 2010 (2010-02-11), pages 75630N-75630N-8, XP055203654, ISSN: 0277-786X, DOI: 10.1117/12.841501**

## Description

### Technical Field

The present technology is related to a fluid analysis device, a fluid analysis method, a program and a fluid analysis system, and specifically, is related to a technology of detecting and analyzing fluid such as a blood flow.

### Background Art

In a medical field, for example, at the time of medical treatment, the flow of fluid such as blood or the like has to be detected in some cases. In PTL 1, a technology of an imaging system that captures a coherent light image of a blood vessel area is disclosed. According to this technology, for example, an internal blood vessel of an organ and blood that covers a living body surface can be distinguished.

The patent US7113817 B1 (WINCHESTER ET AL, 2006-09-26) discloses the features in the preambles of claims 1 and 14. The disclosed apparatus and method relate to speckle imaging of blood flow. The exposure time of the camera used for the imaging is optimized for maximizing the speckle contrast.

### Citation List

### Patent Literature

PTL 1: Japanese Unexamined Patent Application Publication No. 2009-136396

### Summary

### Technical Problem

However, there has been a demand of enhancing the detection sensitivity of the fluid such as the blood flow in the imaging system in the related art described above.

Therefore, a main object of the disclosure is to provide a fluid analysis device, a fluid analysis method, a program, and a fluid analysis system that can enhance the detection accuracy of a flow of fluid.

### Solution to Problem

The invention is defined in claims 1 and 14, preferred embodiments in the dependent claims.

### Advantageous Effects of Invention

According to the present disclosure, the detection accuracy of the flow of the fluid can be enhanced. Further, the effects described herein are examples, are not intended to limit the disclosure, and may be any one of effects described in the disclosure.

### Brief Description of Drawings

[fig.1] Fig. 1 is a diagram schematically illustrating a configuration example of a fluid analysis device 1 of a first embodiment of the disclosure.
[fig.2]Fig. 2 is a diagram schematically illustrating a configuration example of another fluid analysis device 1 of the first embodiment.
[fig.3]Fig. 3 is a diagram illustrating a state of forming an image with light using an image forming optical system 3.
[fig.4A]Fig. 4A is a diagram illustrating an example of a speckle image.
[fig.4B]Fig. 4B is a diagram illustrating an example of a speckle image.
[fig.5]Fig. 5 is a diagram illustrating a state of mapping the speckle image.
[fig.6]Fig. 6 is a graph illustrating a relationship between a numerical aperture and speckle contrast.
[fig.7]Fig. 7 is a graph illustrating a relationship between the numerical aperture and a difference between the speckle contrast of the blood flow X and speckle contrast other than that of the blood flow X.
[fig.8]Fig. 8 is a diagram schematically illustrating a configuration example of a fluid analysis device 11 of the modification example of the first embodiment.
[fig.9A]Fig. 9A is a diagram illustrating a configuration of a tool 100 that circulates the fluid.
[fig.9B]Fig. 9B is a diagram illustrating a configuration of a tool 100 that circulates the fluid.
[fig.9C]Fig. 9C is a diagram illustrating a configuration of a tool 100 that circulates the fluid.
[fig.10A]Fig. 10A is a diagram illustrating an example of the speckle image.
[fig.10B]Fig. 10B is a diagram illustrating an example of the speckle image.
[fig.10C]Fig. 10C is a diagram illustrating an example of the speckle image.
[fig.10D]Fig. 10D is a diagram illustrating an example of the speckle image.
[fig.11]Fig. 11 is a graph illustrating a relationship between the numerical aperture and the speckle contrast with respect to the speckle images illustrated in Figs. 10A to 10D.
[fig.12]Fig. 12 is a graph illustrating a relationship between the numerical aperture and the difference between the speckle contrast of the blood flow X and the speckle contrast other than that of the blood flow X with respect to the speckle images illustrated in Figs. 10A to 10D.
[fig.13]Fig. 13 shows a diagram of a medical imaging system, specifically, an endoscopy system, according to some embodiments.

**Description of Embodiments**

Hereinafter, embodiments of the disclosure are described in detail with reference to the accompanying drawings. Further, the disclosure is not limited to the embodiments described below. In addition, the descriptions are presented as follows.

1. First embodiment

(Example of fluid analysis device for adjusting numerical aperture based on speckle data)

2. Second embodiment

(Example of fluid analysis system adjusting numerical aperture based on speckle data)

(1. First embodiment)

<Configuration of fluid analysis device 1>

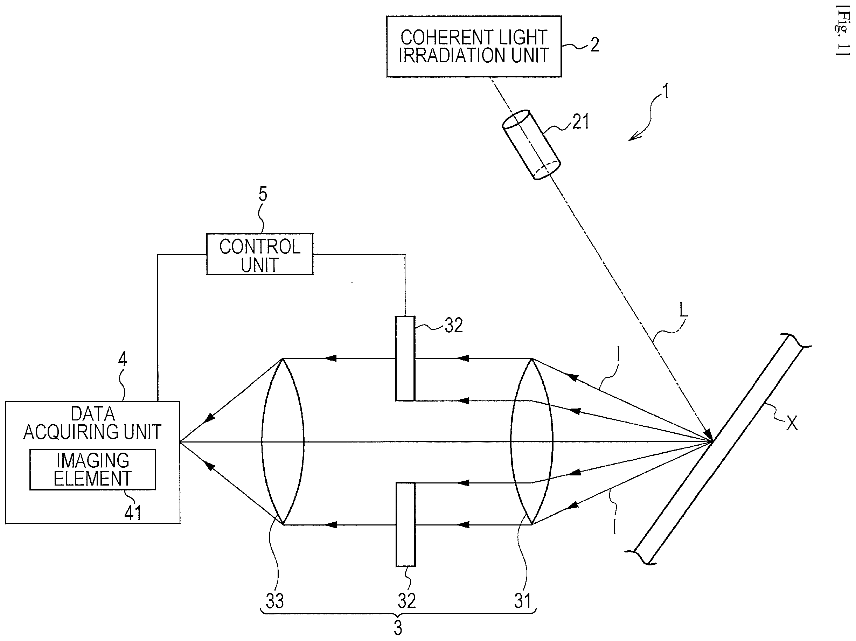

First, a fluid analysis device 1 of the first embodiment of the disclosure is described. Fig. 1 is a diagram schematically illustrating the fluid analysis device 1 of the embodiment. The fluid analysis device 1 of the embodiment includes a coherent light irradiation unit 2 that irradiates the fluid X with coherent light L, an image forming optical system 3 that forms an image with light applied to the fluid X, and a data acquiring unit 4 that acquires the speckle data of the fluid, and the image forming optical system 3 adjusts the numerical aperture based on the speckle data.
In addition, in the fluid analysis device 1 of the embodiment, the fluid X is not particularly limited as long as it has the light scattering material. However, it is preferable that blood is used. At this point, the fluid analysis device 1 functions as a blood flow analysis device, so that while performing medical treatment on an animal such as a human, the fluid analysis device 1 can detect the blood flow thereof and analyze the blood flow. Hereinafter, the fluid X is generally described as the blood flow X.

<Coherent light irradiation unit 2>

The coherent light irradiation unit 2 included in the fluid analysis device 1 of the embodiment irradiates the blood flow X with the coherent light L. The coherent light irradiation unit 2 can be used as a light source for irradiation with the coherent light L. As described below, the coherent light L is not particularly limited as long as the speckle data can be obtained from light applied to the blood flow X, and a laser beam can be used as the coherent light L. In addition, a beam expander 21 can be provided between the coherent light irradiation unit 2 and the blood flow X.

<Image forming optical system 3>

The image forming optical system 3 included in the fluid analysis device 1 of the embodiment forms an image with the light applied to the blood flow X. In addition, the image forming optical system 3 adjusts the numerical aperture based on the speckle data acquired by the data acquiring unit 4 as described below. It is preferable that the speckle data is data of the speckle contrast, and at this point, the image forming optical system 3 adjusts the numerical aperture so that the speckle contrast becomes the maximum. Accordingly, the detection accuracy of the flow of the blood flow X can be enhanced. In addition, the details of the speckle contrast are described below.

The image forming optical system 3 mainly includes a first lens 31 that functions as a condensing lens that focuses the light 1 applied to the blood flow X, a diaphragm 32 that can change the opening diameter in a plane direction parallel to a Fourier surface, and a second lens 33 that functions as an imaging lens focusing on the imaging device 41 described below. The image forming optical system 3 can adjust the numerical aperture by adjusting the size of the diaphragm 32.

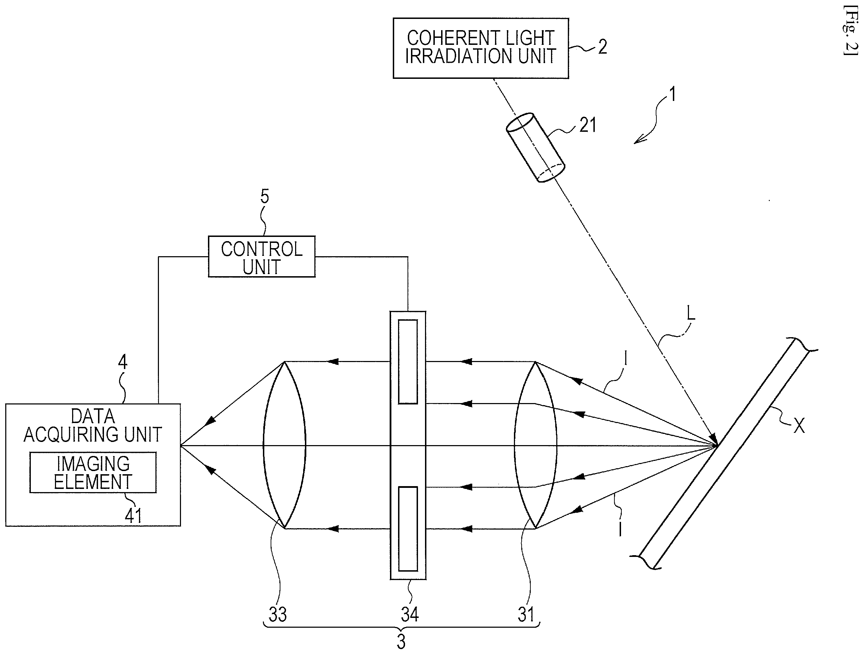

Fig. 2 is a diagram illustrating a concept of another device configuration of the fluid analysis device 1. As illustrated in Fig. 2, the image forming optical system 3 may have an optional spatial optical modulator 34, instead of the diaphragm 32. In the fluid analysis device 1 of the embodiment, the speckle contrast of the speckle image can be caused to be the maximum by controlling the opening of the spatial optical modulator 34, instead of adjusting the size of the diaphragm 32.

In addition, the fluid analysis device 1 of the embodiment can be operated by using a digital micro device (DMD). Further, at the position of the diaphragm 32 described above, an optional light valve that can electronically or mechanically control the numerical aperture can be provided. As a device that causes the image forming optical system 3 to change the numerical aperture, an optional optical modulating element such as a mechanical iris diaphragm, a liquid crystal shutter, an electrochromic device, and an electrophoretic device can be used.

<Data acquiring unit 4>

The data acquiring unit 4 included in the fluid analysis device 1 of the embodiment acquires the speckle data of the blood flow X irradiated with the coherent light L. Particularly, the data acquiring unit 4 can include the imaging device 41as a CCD camera, and it is possible to capture the speckle image of the blood flow X with the imaging device 41 and to acquire the speckle data.

The data acquiring unit 4 can transmit a signal relating to the acquired speckle data to a control unit 5 described below, and adjusts the numerical aperture of the image forming optical system 3 by the control unit 5. Also, in the fluid analysis device 1 of the embodiment, in order to statistically process a speckle pattern described below and to accurately obtain data of the speckle contrast with respect to the numerical aperture, a pixel size of the image captured by the imaging device 41 is preferably smaller than a speckle grain size.

<Control unit 5>

The fluid analysis device 1 of the embodiment can further include the control unit 5. In the control unit 5, a central processing unit (CPU), a memory, an input and output interface unit, a hard disk or the like may be provided. Details thereof are described in the fluid analysis method described below. However, the control unit 5 can generate the speckle data for each numerical aperture based on the speckle data acquired by the data acquiring unit 4. Specifically, the control unit 5 can generate data of the speckle contrast for each numerical aperture. Also, the control unit 5 can adjust the numerical aperture of the diaphragm 32 in the image forming optical system 3 based on the speckle data acquired by the data acquiring unit 4.

<Fluid analysis method>

Next, an example of the fluid analysis method of the blood flow X performed by the fluid analysis device 1 of the embodiment is described. The fluid analysis method includes a data acquiring step of irradiating the blood flow X with coherent light and acquiring speckle data of the fluid irradiated with the coherent light and a step of adjusting the numerical aperture of the image forming optical system 3 for forming an image with the light applied to the fluid based on the speckle data.

In the fluid analysis device 1 of the embodiment, the numerical aperture of the diaphragm 32 of the image forming optical system 3 is adjusted to an optimum value by the control unit 5 so that the movement of the scattered fluid of the blood flow X can be most distinctly observed from the speckle data acquired by the data acquiring unit 4. Particularly, the image forming optical system 3 adjusts the numerical aperture so that the speckle contrast becomes the maximum.

<Speckle data>

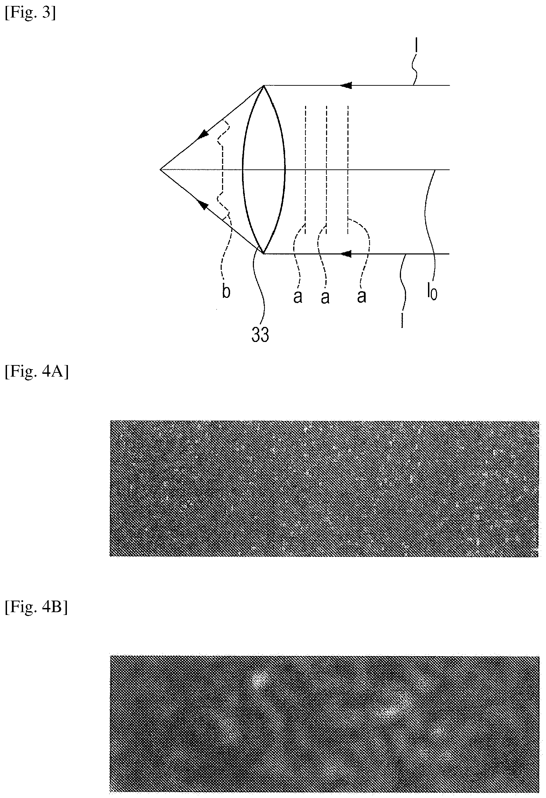

Here, the speckle data for adjusting the numerical aperture is described more specifically. Fig. 3 is a diagram illustrating a state of forming an image with light using the image forming optical system 3. As illustrated in Fig. 3, rays near an optical axis $l_0$ of the light applied to the blood flow X are not disturbed on the wave surface, but the rays are disturbed on the wave surface as it gets farther from the optical axis (in the drawing, see references a and b), and a speckle phenomenon occurs due to the random interference of the scattered wave. Accordingly, the imaging device 41 can capture a spot pattern image (speckle image) of the blood flow X.

At this point, the image forming optical system 3 causes the numerical aperture to be great so that angles and directions of the incident light and phases vary and the scattered waves are equalized. Therefore, the speckle contrast decreases. Meanwhile, when the image forming optical system 3 causes the numerical aperture to be small, the speckle contrast becomes great. Further, if the wave front aberration in the image forming optical system 3 becomes small, the main phase difference occurs due to the scatter from a rough surface of the blood flow X. At this point, if the phase difference applied by the scatter from the blood flow X is in a range of 1 cycle (-pi to pi), a phenomenon called "undeveloped speckle" by the image forming optical system 3 is generated so that a speckle pattern changes and the speckle contrast decreases on the imaging surface.

If the blood flow X is detected by using the change of the speckle data, it is considered that when the speckle contrast is great, the flow measurement sensitivity or the accuracy becomes high. Therefore, if the spatial resolution is negligible, the flow can be measured with high accuracy by reducing the numerical aperture of the image forming optical system 3 to be as small as possible in a range in which the speckle is not undeveloped.

The numerical aperture at this point varies in response to the phase difference occurring due to the blood flow X which is a measuring object, the aberration of the image forming optical system 3, the numerical aperture of the illumination system, and the sensitivity of the two-dimensional imaging device 41. The image forming optical system 3 changes the numerical aperture based on the speckle data (intensity of speckle contrast, speckle pattern, or the like) acquired by capturing the speckle image by the data acquiring unit 4.

As described above, as a device for adjusting the numerical aperture of the image forming optical system 3 based on the signal from the speckle data obtained from the control unit 5, an optional optical modulating element such as a mechanical iris diaphragm, a liquid crystal shutter, an electrochromic device, and an electrophoretic device can be used.

Subsequently, with reference to Figs. 4A and 4B, the aforementioned undeveloped speckle and the sufficiently developed speckle are described below. Figs. 4A and 4B are diagrams illustrating an example of the speckle image.

<Sufficiently developed speckle>

In general, a complex amplitude A at an optional observation point x on an image surface is given by Expression (1) below (see Speckle phenomena in optics: theory and applications, Chapter 2, pp. 7-23, Roberts & Company, Englewood, Colorado, written by J. W. Goodman).
[Math.1]

$$A(x) = |A(x)| \cdot \exp[i\theta(x)] \qquad \text{Expression (1)}$$

In addition, in Expression (1), theta represents a phase.

A complex amplitude of the light made on an image surface by n points of a dispersed object such as the blood flow X is expressed by Expression (2) below.
[Math.2]

$$A(x) = \sum_{k=1}^{N} \frac{1}{\sqrt{N}} a_k(x) = \frac{1}{\sqrt{N}} \sum_{k=1}^{N} |a_k(x)| \cdot \exp(i\phi_k) \qquad \text{Expression (2)}$$

If the phase distribution of each light from N scatter points of the scattered object independently is the same, the probability density function with respect to a real portion Ar and an imaginary portion Ai of the complex amplitude is expressed by Expression (3) from the central limit theorem.
[Math.3]

$$P_{r,i}\left(A_r, A_i\right) = \frac{1}{2\pi\sigma^2}\exp\left(-\frac{A_r^2 + A_i^2}{2\sigma^2}\right)$$

Expression (3)

Further, in Expression (3), sigma represents a standard deviation between Ar and Ai.

At this point, intensity I and a phase theta of the speckle are expressed by Expressions (4) and (5) below, respectively. Therefore, an intensity probability density function $P_I(I)$ and a phase probability density function $P_{theta}(theta)$ are expressed by Expressions (6) and (7) below, respectively.

[Math.4]

$$I = A_r^2 + A_i^2$$

Expression (4)

[Math.5]

$$\theta = \tan^{-1}\left(\frac{A_i}{A_r}\right)$$

Expression (5)

[Math.6]

$$P_I(I) = \frac{1}{2\sigma^2}\exp\left(-\frac{I}{2\sigma^2}\right)$$

Expression (6)

[Math.7]

$$P_\theta(\theta) = \frac{1}{2\pi}$$

Expression (7)

The speckle expressed by the probability density functions can be called a sufficiently developed speckle, and a pattern illustrated in Fig. 4A is an example thereof.

<Undeveloped speckle>

Meanwhile, if the phase is in a range of -pi <= theta <= pi, and the distribution is not even, the probability density function is expressed by a general Gauss probability density function of Expression (8) below.

[Math. 8]

$$P_{r,i}\left(A_r, A_i\right) = \frac{1}{2\pi\sigma_r\sigma_i\left(1-\rho^2\right)^{1/2}} \cdot \exp\left[-\frac{1}{\left(1-\rho^2\right)} \cdot \left(\frac{\Delta A_r^2}{\sigma_r^2} - \frac{2\rho \cdot \Delta A_r \cdot \Delta A_i}{\sigma_r \cdot \sigma_i} + \frac{\Delta A_i^2}{\sigma_i^2}\right)\right]$$

Expression (8)

Further, in Expression (8), rho is a correlation coefficient between Ar and Ai, delta Ar = Ar - <Ar>, and delta Ai = Ai - <Ai>.

The speckle expressed by the probability density function is called an undeveloped speckle, and a pattern illustrated in Fig. 4B is an example.

<Speckle contrast>

The control unit 5 can convert information of the blood flow X into the speckle contrast by performing data processing on the speckle data acquired by the data acquiring unit 4. The speckle contrast (CS) is expressed by Expression (9) below. In addition, the velocity of the light scattered fluid such as the blood flow X is inversely proportional to a square of the speckle contrast. (See Opt. Commun. 37 (5), p. 325 (1981) or the like)
[Math.9]

$$C_S = \frac{\sigma}{\langle I \rangle}$$

Expression (9)

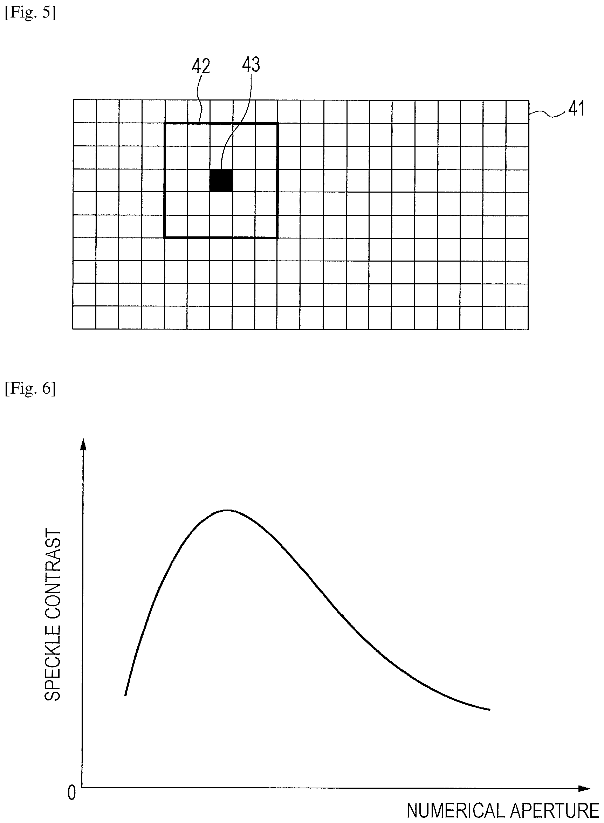

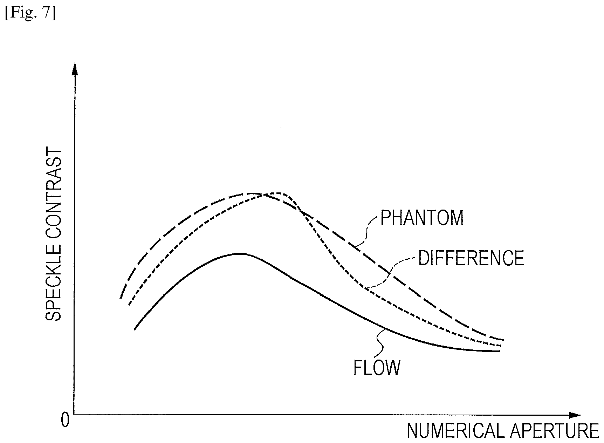

Fig. 5 is a diagram schematically illustrating a state of mapping the speckle image. With respect to the speckle image illustrated in Figs. 4A and 4B, the entire range of an image 41 for each pixel is mapped as illustrated in Fig. 5 so that the image of the blood flow X can be generated, and the movement and the flow of the blood flow X can be observed.
As illustrated in Fig. 5, for example, an area of the image 41 in which statistical processing for calculating the speckle contrast is performed is set to be a square area 42 in which five pixels are arranged respectively in horizontal and vertical directions. For example, the speckle contrast in a center 43 of the square area 42 can be calculated as the speckle contrast of the square area. An optional range can be set as the statistical processing area depending on the resolution of the speckle image and the measurement accuracy of the flow of the blood flow X.
Fig. 6 is a graph illustrating a relationship between the numerical aperture and the speckle contrast. In the graph illustrated in Fig. 6, the image forming optical system 3 can adjust the numerical aperture so that the speckle contrast becomes the maximum. Accordingly, the detection accuracy of the flow of the blood flow X can be enhanced.
The data acquiring unit 4 acquires data of speckle contrast other than that of the fluid such as the blood flow X, and the image forming optical system 3 can adjust the numerical aperture so that the speckle contrast of the blood flow X and the speckle contrast other than that of the blood flow X becomes the maximum. Fig. 7 is a graph illustrating a relationship between the numerical aperture and the speckle contrast. Particularly, Fig. 7 is a graph illustrating the numerical aperture and the difference between the speckle contrast of the blood flow X and the speckle contrast other than that of the blood flow X. In the present invention, the detection accuracy of the flow of the blood flow X is increased by adjusting the numerical aperture so that the difference (difference in Fig. 7) between the speckle contrast of the blood flow X (flow in Fig. 7) and the speckle contrast other than that of the blood flow X (phantom in Fig. 7) becomes the maximum.
In the above, the method of adjusting the numerical aperture so that the speckle contrast becomes the maximum is described, but the image forming optical system 3 may adjust the numerical aperture based on the granularity or the stripe density of the speckle image. With reference to Figs. 4A and 4B, the speckle image illustrated in Fig. 4A has granularity greater than that of the speckle image illustrated in Fig. 4B. Meanwhile, the speckle image illustrated in Fig. 4B has stripe density greater than that of the speckle image illustrated in Fig. 4A. The control unit 5 can control the numerical aperture in response to the value of the granularity and stripe density thereof, by distributing, for example, how much pixels having luminance values which are the same or in a certain range are continued and adjacent to each other. In this manner, the image forming optical system 3 can adjust the numerical aperture so that the granularity of the speckle image becomes greater.
As described above, since in the fluid analysis device 1 of the embodiment, the image forming optical system 3 adjusts the numerical aperture based on the speckle data, it is possible to enhance the detection accuracy of the flow of the blood flow X. Particularly, the blood flow X can be accurately observed by causing the image forming optical system 3 to adjust the numerical aperture so that the speckle contrast becomes the maximum by using the data of the speckle contrast as the speckle data. In the present invention, the blood flow X can be accurately observed by causing the image forming optical system 3 to adjust the numerical aperture so that the difference between the speckle contrast of the blood flow X and the speckle contrast other than that of the blood flow X becomes the maximum.

(Modification example)

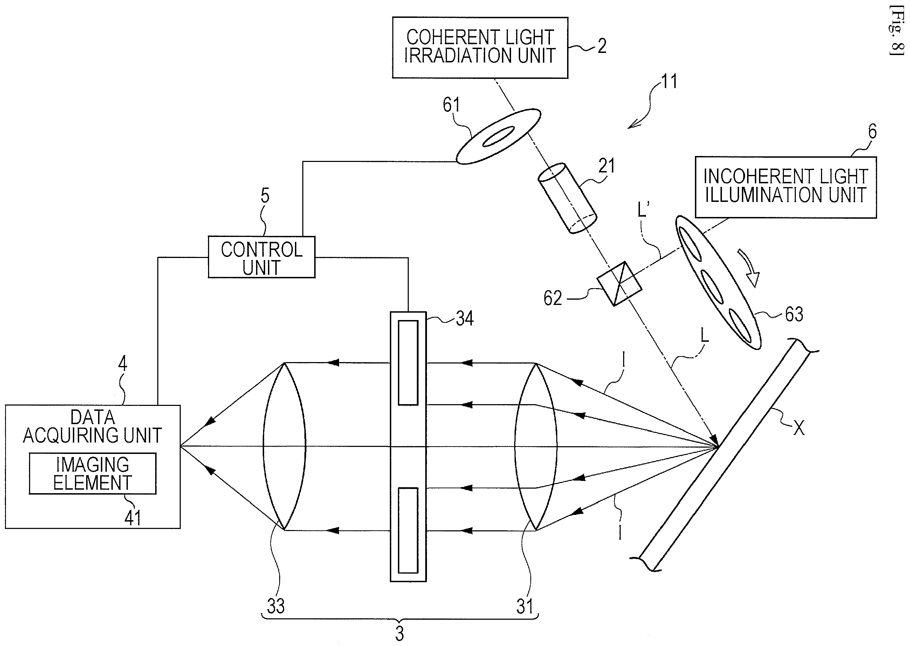

Fig. 8 is a diagram schematically illustrating a configuration example of the fluid analysis device 11 of the modification example of the embodiment. The fluid analysis device 11 of the modification example of the embodiment is different from the fluid analysis device 1 of the first embodiment described above in that the fluid analysis device 11 includes an incoherent light irradiation unit 6 that irradiates the blood flow X with incoherent light L'. Therefore, herein, the configuration of the incoherent light irradiation unit 6 and the function thereof are mainly described.

As illustrated in Fig. 8, when a bright field image or a fluorescence image is observed by irradiating the blood flow X with the incoherent light L by the incoherent light irradiation unit 6, and using the image forming optical system 3 which is the same as the coherent light irradiation unit 6, a necessary resolution and brightness are different. Therefore, the image forming optical system 3 can adjust the diaphragm 32 so that the size of the aperture becomes optimal for the speckle image and the bright field and fluorescence image. As the incoherent light irradiation unit 6 is not particularly limited, a visible light laser with low coherency such as a Xe lamp can be used.

Further, in the fluid analysis device 11 of this modification example, a rotating chopper 61 may be provided between the coherent light irradiation unit 2 and the beam expander 21. In addition, a polarization beam splitter 62 may be provided between the beam expander 21 and the blood flow X. For example, the parallel light from the Xe lamp can pass through a rotating band pass filter 63 that can block the red light, the green light, and the blue light at an even time interval, and be incident to and reflected on the polarization beam splitter 62. The rotating chopper 61 and the rotating band pass filter 63 can be synchronized, and the same position of the blood flow X can be illuminated with red, green, and blue naturally emitted light and a near infrared laser beam at a specific time interval.

In addition, the configurations and the effects of the fluid analysis system of the embodiment in addition to the above are the same as in those in the first embodiment.

(2. Second embodiment)

Subsequently, the fluid analysis system according to the second embodiment of the disclosure is described. In the fluid analysis system of the second embodiment, the control unit 5 can be provided in a different device from the fluid analysis devices 1, and 11, compared with the fluid analysis device 1 of the first embodiment and the fluid analysis device 11 of the modification example of the first embodiment. Accordingly, for example, the control unit 5 can adjust the numerical aperture based on the speckle data in the image forming optical system 3 of the fluid analysis device 1 via a network.

The network includes, for example, a public network such as the Internet, a telephone network, a satellite communication network, and a broadcast communication path, or a leased line network such as a wide area network (WAN), a local area network (LAN), an internet protocol-virtual private network (IP-VPN), an Ethernet (registered trade mark), and a wireless LAN, regardless of being wired or wireless. In addition, the network may be a communication channel network exclusively provided in the fluid analysis system of the embodiment.

Also, a server, an image display apparatus, or the like may be provided in the fluid analysis system of the embodiment. In this case, the fluid analysis devices 1, and 11, a server, and an image display apparatus may be directly connected, or may be connected in a communicable manner via a network.

In addition, the configurations and the effects of the fluid analysis system of the embodiment in addition to the above are the same as in those in the first embodiment.

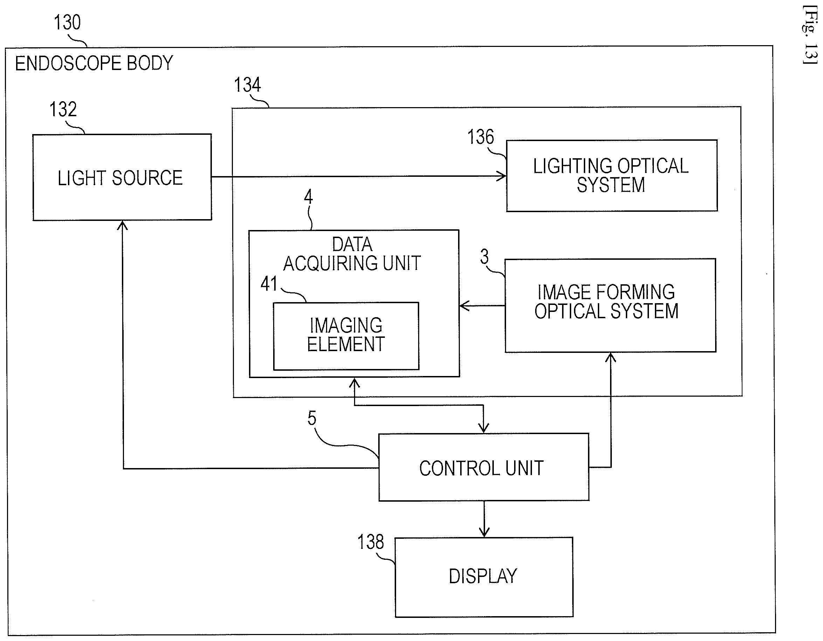

Fig. 13 shows a diagram of a medical imaging system, specifically, an endoscopy system 130, according to some embodiments. As shown in FIG. 13, the endoscopy system 130 has a light source 132. Light source 132 may include a coherent light irradiation unit 2, an incoherent light illumination unit 6, or both a coherent light irradiation unit 2, an incoherent light illumination unit 6, as discussed above. The light source 132 emits light that is provided to an endoscope body 134 to perform endoscopy.

The endoscope body 134 may include a lighting optical system 136 to receive the light from light source 132. The lighting optical system 136 may include any suitable optical components, such as one or more lenses, one or more optical fibers, a beam expander 21, a beam splitter such as polarization beam splitter 62, a filter such as rotating band pass filter 63, or any suitable combination of such components. However, the techinques described herein are not limited to the lighting optical system 136 being included within the endoscope body 134, as in some embodiments on or more components of the lighting optical system 136 may be provided external to the endoscope body 134. Light may be emitted from the lighting optical system to 136 to illuminate a region of an organism, such as the human body, to perform endoscopy.

The endoscope body 134 may also include an image forming optical system 3 and a data acquiring unit 4 which may include an imaging element 41, as described above.

The endoscopy system 130 may include a control unit 5 that receives data from the data acquiring unit 4 and controls the image forming optical system 3. The control unit 5 and/or the data acquiring unit 4 may include a processor that processes the received image data to obtain speckle data, such as a speckle contrast. In some embodiments, the control unit 5 may control the light source 132.

The endoscopy system 130 may include a display 138 for display of an image produced by the data acquiring unit 4 and/or the control unit 5. Display 138 may be any suitable type of display for displaying images to a user.

In some embodiments, a medical imaging system may include a microscope including an optical apparatus as described above and shown in FIG. 1, by way of example.

**Examples**

Effects of the disclosure are described in detail with reference to examples of the disclosure.

**Example 1**

Fluid is observed using the fluid analysis device illustrated in Fig. 1. Specifically, an external resonance semiconductor laser (CW oscillation, wavelength: 780 nanometer, single vertical mode, line width: 300 kHz (1 ms), transverse mode: TEM00, output number: up to 100 mW) manufactured by Sacher Lasertechnik is used as a light source. Also, a laser beam emitted from the light source is expanded by a beam expander manufactured by Edmund Optics, and a phantom formed as a channel is irradiated with a parallel beam.

In a structure of the phantom, alumina particles as a light diffusion agent and red ink as a light absorbent are mixed in an ultraviolet curing resin, and an absorption coefficient and an equivalent scatter coefficient in a wavelength of 780 nanometer are set to be 0.07 per millimeter and 1.08 per millimeter so as to be the same as those of an inner wall of a stomach of a human.

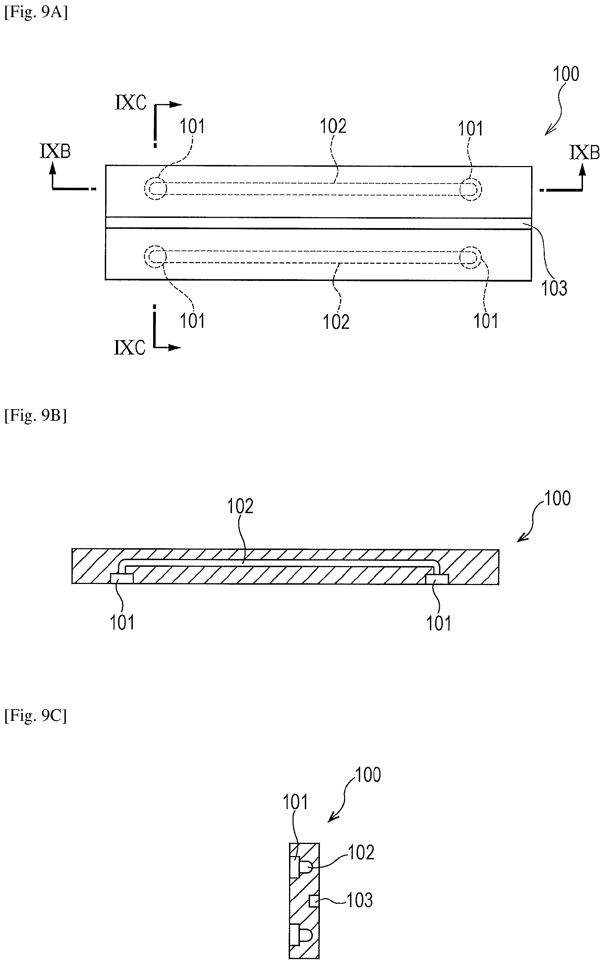

Figs. 9A to 9C are diagrams illustrating a configuration of the tool 100 circulate the fluid. A cross-sectional view taken along line IXB-IXB in a front view of Fig. 9A is illustrated in Fig. 9B, and a cross-sectional view taken along line IXC-IXC is illustrated in Fig. 9C. Specifically, as illustrated in Figs. 9A to 9C, an opening portion 101 and a channel 102 of which a cross section has a 1 millimeter angle are formed in positions having a depth of 200 micrometers from an observation surface, and a tube is connected to an injection and discharge port provided on a rear side with respect to the observation surface. In addition, artificial blood flows from the opening portion 101 to the channel 102 at a velocity of about 10 millimeter/sec with a syringe pump. In Example 1, drinkable milk is used as the artificial blood. A reference number 103 denotes a focusing member.

Specifically, two sheets of spherical planoconvex quartz lenses having a focal length of 150 millimeter and an opening diameter of 30 millimeter are used as the image forming optical system 3, the diaphragm 32 (iris diaphragm) is arranged on a Fourier surface therebetween, and a diameter of the aperture is adjustable in a range of 3 millimeter to 15 millimeter.

A focus is adjusted so that an imaging surface of a CCD camera is identical to an image surface of the data acquiring unit 4. An industrial CCD camera (XCD-V60) manufactured by Sony Corporation is used as an imaging CCD camera. In addition, the camera has a pixel size sufficiently smaller than a speckle grain size so that an intensity of a speckle pattern is a resolution sufficient for statistical processing (square of which one side is 7.4 micrometers).

A signal from the CCD camera is output as a bitmap file of 8 bits, VGA, 60 fps, and is obtained by a PC according to IEEE1394b, to perform statistical processing. An image of the flow is generated by setting an image area for performing statistical processing in order to calculate speckle contrast as a square area having five pixels respectively in horizontal and vertical directions, setting speckle contrast at this point as speckle contrast in the center of the area, and mapping the entire image.

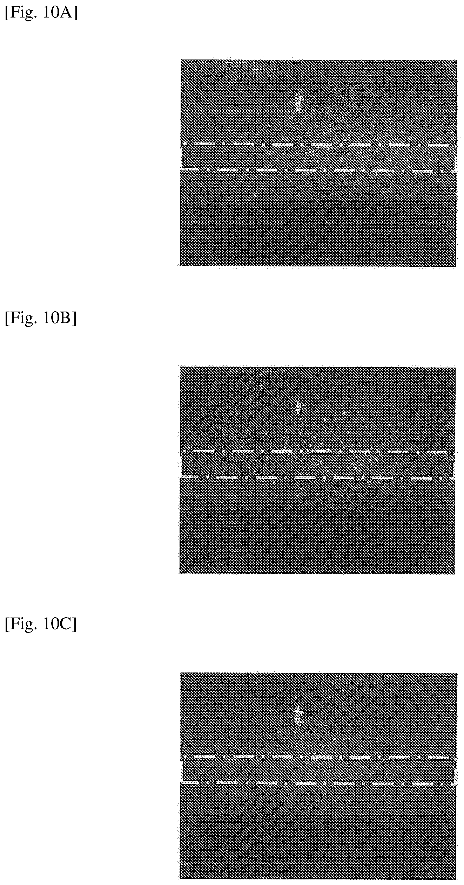

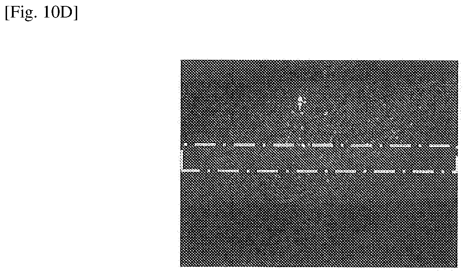

Figs. 10A to 10D are diagrams illustrating an example of the speckle image obtained in this manner. As illustrated in Figs. 10A to 10D, portions enclosed with frames of alternate long and short dashed lines in all patterns correspond to channels. Figs. 10A and 10B illustrate speckle when the flow of the fluid X is stopped, and Figs. 10C and 10D illustrate the fluid X flowing. Also, Figs. 10A and 10C illustrate that the opening diameter of the iris diaphragm is 12 millimeter, and Figs. 10B and 10D illustrate that the opening diameter is 4 millimeter. It can be observed that speckle patterns and contrast become different according to whether the fluid flows or not or according to the size of the aperture, from the image illustrated in Figs. 10A to 10D.

From the image information, the speckle contrast is calculated in the method above with reference to Expression (9). Particularly, the speckle contrast for each size of the aperture can be obtained by obtaining a standard deviation sigma and an average <I> from the frequency distribution of the luminance gradation obtained by subtracting a value corresponding to a dark current for each pixel with respect to the area in which the illuminance near the center of the channel image is even.

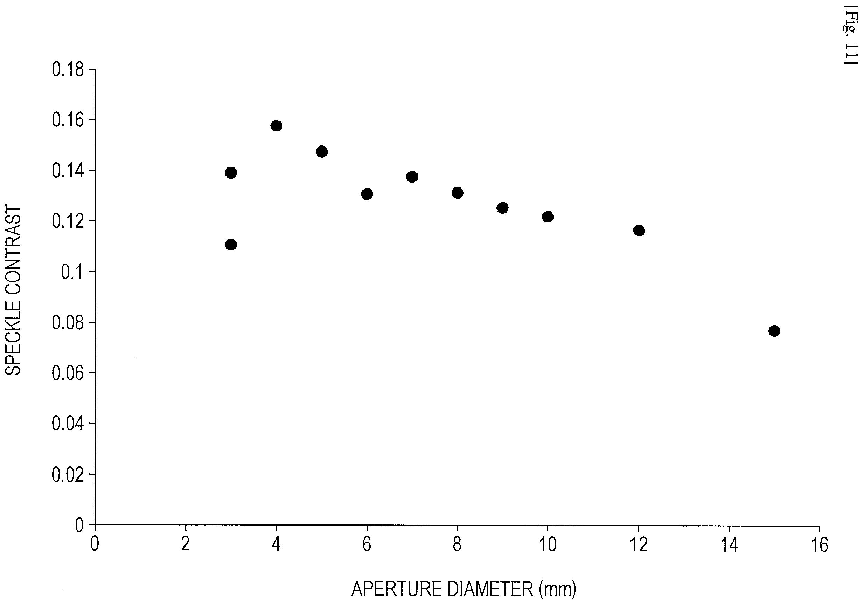

Fig. 11 is a graph illustrating a relationship between the numerical aperture and the speckle contrast with respect to the speckle images illustrated in Figs. 10A to 10D. As illustrated in Fig. 11, in the optical system and the sample according to Example 1, it is found that when the size of the aperture is 4 millimeter, the speckle contrast is obtained as a maximum value. That is, it is found that the sensitivity and the accuracy become the maximum by setting the numerical aperture of the image forming optical system 3 to be 4 millimeter in order to visualize the flow of the fluid. In this manner, the numerical aperture of the image forming optical system 3 can be controlled so that the speckle contrast becomes the maximum.

## Example 2

In Example 2, a test is performed in the same manner as in Example 1 except for controlling the aperture so that the difference between the speckle contrast and speckle contrast other than that of the fluid X becomes the maximum instead of controlling the aperture so that the speckle contrast becomes the maximum.

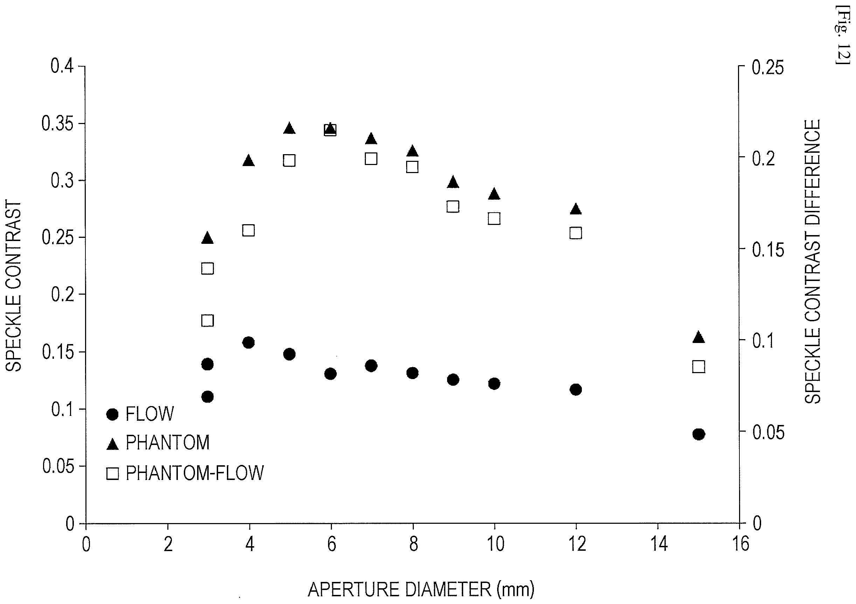

Fig. 12 is a graph illustrating a relationship between the numerical aperture and the difference between the speckle contrast and the speckle contrast other than that of the fluid X with respect to the speckle images illustrated in Figs. 10A to 10D. As illustrated in Fig. 12, in Example 2, it is found that the sensitivity and the accuracy become the maximum by setting the size of the aperture of the image forming optical system 3 to be about 6 millimeter. In this manner, the numerical aperture of the image forming optical system 3 can be controlled.

## Example 3

A test is performed in the same manner as in Example 1 except for using the image forming optical system 3 illustrated in Fig. 2 instead of the image forming optical system 3 used in Examples 1 and 2. Specifically, an image of the flow is captured by controlling the aperture in real time by the liquid crystal transmission-type spatial optical modulator manufactured by Holoeye photonics installed in substitution for the iris diaphragm. In addition, the size of the aperture of which a speckle pattern changes from a particle shape to a stripe shape is obtained while the aperture of the spatial optical modulator is controlled, and the image of the flow with the numerical aperture and the shape thereof is captured. Accordingly, the test result which is the same as that in Example 1 can be obtained.

## Example 4

A test is performed in the same manner as in Examples 1, 2, and 3 except for using the fluid analysis device 11 illustrated in Fig. 8 instead of the fluid analysis device 1 used in Examples 1, 2, and 3. Particularly, based on an optical path of Example 1 or the like, the rotating chopper 61 is provided in front of the beam expander 21, and the broad band polarization beam splitter 62 which is valid when the wavelength is in the range of 400 nanometer to 800 nanometer is provided behind the beam expander 21. The polarization beam splitter 62 is arranged so as to be penetrated by a laser in the wavelength of 780 nanometer. Also, the parallel light from the Xe lamp can pass through the rotating band pass filter 63 in which the blockage of red, green, and blue changes at an even time interval, and be incident to and reflected on the polarization beam splitter 62. The rotating chopper 61 and the rotating band pass filter 63 are synchronized, and the same position on the sample surface can be illuminated with red, green, and blue naturally emitted light and a near infrared laser beam at the same time interval. The rotating chopper 61 and the rotating band pass filter 63 are synchronized with an imaging camera (the imaging device 41), the images illuminated with the red, green, and blue light and a near infrared laser beam are repeatedly obtained, and the image data is sent to a PC.

In the diaphragm on a Fourier surface, the spatial optical modulator 34 which is the same as in Example 3 is provided. When the spatial resolution is necessary for bright field observation, the numerical aperture is set to be great, and when the sensitivity and the accuracy of the flow in the speckle image are necessary, the numerical aperture is set so that the sensitivity and the accuracy of the flow detection in the method of Example 2 become high, and this aperture control is repeated. Accordingly, a good bright field image and a good image of a flow in the same observation position can be simultaneously observed in real time. In addition, in Example 4, the Xe lamp is used as the incoherent light irradiation unit 6, but a visible light laser with sufficiently low coherency may be used.

In Example 4, it is found that optimum sensitivity, accuracy, resolution, and a focal depth are obtained when the image forming optical system is used together with the bright field image, the fluorescence image, and the like.

In the above, in Examples 1 to 4, it can be found that the blood flow is accurately observed by adjusting the numerical aperture of the image forming optical system in the fluid analysis device of the disclosure.

It should be understood by those skilled in the art that various modifications, combinations, sub-combinations and alterations may occur depending on design requirements and other factors insofar as they are within the scope of the appended claims or the equivalents thereof.

## Reference Signs List

1, 11 Fluid analysis device
2 Coherent light irradiation unit
3 Image forming optical system
4 Data acquiring unit

5 Control unit
6 Incoherent light irradiation unit
31 First lens
32 Diaphragm
33 Second lens
X Fluid (blood flow)

**Claims**

1. An imaging apparatus (1), comprising:

   a light source (2) that is configured to emit coherent light to image an object comprising a fluid;
   an optical apparatus (3) that is configured to optically process light from the object, the optical apparatus having a numerical aperture;
   an imager (4) that is configured to receive the light optically processed by the optical apparatus; and
   circuitry (5) configured to obtain speckle data based on the light received by the imager and control the numerical aperture of the optical apparatus based on the speckle data; the said apparatus is **characterized in that** the circuitry is configured to control the numerical aperture of the optical apparatus to increase a difference between speckle data of the fluid and speckle data of a non-fluid.

2. The imaging apparatus of claim 1, wherein the speckle data is a speckle contrast.

3. The imaging apparatus of claim 2, wherein the circuitry is configured to control the numerical aperture of the optical apparatus to increase the speckle contrast.

4. The imaging apparatus of claim 3, wherein the circuitry is configured to control the numerical aperture of the optical apparatus to maximize the speckle contrast.

5. The imaging apparatus of claim 1, wherein the circuitry is configured to produce image data based on the speckle data, the image data indicating a location of the fluid.

6. The imaging apparatus of claim 1, wherein the speckle data is a speckle contrast and the circuitry is configured to control the numerical aperture of the optical apparatus to maximize a difference between the speckle contrast of the fluid and speckle contrast of a non-fluid.

7. The imaging apparatus of claim 1, wherein the fluid comprises blood and the imaging apparatus images blood.

8. The imaging apparatus of claim 1, wherein the circuitry comprises a controller.

9. The imaging apparatus of claim 1, wherein the imager has a pixel size smaller than a speckle grain size of the light from the object.

10. The imaging apparatus of claim 1, wherein the light source is a first light source and the imaging apparatus further comprises:

    a second light source that emits incoherent light to image the object,
    wherein the imager images the object using the incoherent light.

11. The imaging apparatus of claim 1, wherein the circuitry is configured to control the numerical aperture of the optical apparatus by changing a size of an opening in the optical apparatus.

12. The imaging apparatus of claim 11, wherein the optical apparatus comprises a diaphragm.

13. The imaging apparatus of claim 11, wherein the optical apparatus comprises a spatial optical modulator.

14. An imaging method, comprising:

emitting coherent light to image an object;
optically processing light from the object using an optical apparatus having a numerical aperture;
obtaining speckle data based on the light optically processed by the optical apparatus; and
controlling the numerical aperture of the optical apparatus based on the speckle data; wherein
the object comprises a fluid; the said method is **characterized in that** the numerical aperture is controlled to increase a difference between speckle data of the fluid and speckle data of a non-fluid.

**15.** A medical imaging system, comprising an imaging apparatus according to claim 1.

**Patentansprüche**

**1.** Bildgebungsvorrichtung (1), umfassend:

eine Lichtquelle (2), die ausgelegt ist zum Emittieren von kohärentem Licht zum Abbilden eines ein Fluid umfassenden Objekts;
eine optische Vorrichtung (3), die ausgelegt ist zum optischen Verarbeiten von Licht von dem Objekt, wobei die optische Vorrichtung eine numerische Apertur aufweist;
einen Bildwandler (4), der ausgelegt ist zum Empfangen des durch die optische Vorrichtung optisch verarbeiteten Lichts; und
eine Schaltungsanordnung (5), die ausgelegt ist zum Erhalten von Speckle-Daten auf Basis des durch den Bildwandler empfangenen Lichts und Steuern der numerischen Apertur der optischen Vorrichtung auf Basis der Speckle-Daten;
wobei die Vorrichtung **dadurch gekennzeichnet ist, dass**
die Schaltungsanordnung ausgelegt ist zum Steuern der numerischen Apertur der optischen Vorrichtung, um eine Differenz zwischen Speckle-Daten des Fluids und Speckle-Daten eines Nicht-Fluids zu vergrößern.

**2.** Bildgebungsvorrichtung nach Anspruch 1, wobei es sich bei den Speckle-Daten um einen Speckle-Kontrast handelt.

**3.** Bildgebungsvorrichtung nach Anspruch 2, wobei die Schaltungsanordnung ausgelegt ist zum Steuern der numerischen Apertur der optischen Vorrichtung, um den Speckle-Kontrast zu erhöhen.

**4.** Bildgebungsvorrichtung nach Anspruch 3, wobei die Schaltungsanordnung ausgelegt ist zum Steuern der numerischen Apertur der optischen Vorrichtung, um den Speckle-Kontrast zu maximieren.

**5.** Bildgebungsvorrichtung nach Anspruch 1, wobei die Schaltungsanordnung ausgelegt ist zum Erzeugen von Bilddaten auf Basis der Speckle-Daten, wobei die Bilddaten einen Ort des Fluids anzeigen.

**6.** Bildgebungsvorrichtung nach Anspruch 1, wobei es sich bei den Speckle-Daten um einen Speckle-Kontrast handelt und die Schaltungsanordnung ausgelegt ist zum Steuern der numerischen Apertur der optischen Vorrichtung, um eine Differenz zwischen dem Speckle-Kontrast des Fluids und dem Speckle-Kontrast eines Nicht-Fluids zu maximieren.

**7.** Bildgebungsvorrichtung nach Anspruch 1, wobei das Fluid Blut umfasst und die Bildgebungsvorrichtung Blut abbildet.

**8.** Bildgebungsvorrichtung nach Anspruch 1, wobei die Schaltungsanordnung einen Controller umfasst.

**9.** Bildgebungsvorrichtung nach Anspruch 1, wobei der Bildwandler eine Pixelgröße aufweist, die kleiner ist als eine Speckle-Korngröße des Lichts von dem Objekt.

**10.** Bildgebungsvorrichtung nach Anspruch 1, wobei die Lichtquelle eine erste Lichtquelle ist und die Bildgebungsvor-rrichtung weiterhin umfasst:

eine zweite Lichtquelle, die inkohärentes Licht zum Abbilden des Objekts emittiert,
wobei der Bildgeber das Objekt unter Verwendung des inkohärenten Lichts abbildet.

**11.** Bildgebungsvorrichtung nach Anspruch 1, wobei die Schaltungsanordnung ausgelegt ist zum Steuern der numerischen Apertur der optischen Vorrichtung durch Ändern einer Größe einer Öffnung in der optischen Vorrichtung.

**12.** Bildgebungsvorrichtung nach Anspruch 11, wobei die optische Vorrichtung eine Blende umfasst.

**13.** Bildgebungsvorrichtung nach Anspruch 11, wobei die optische Vorrichtung einen räumlichen optischen Modulator umfasst.

**14.** Bildgebungsverfahren, umfassend:

Emittieren von kohärentem Licht, um ein Objekt abzubilden;
optisches Verarbeiten von Licht von dem Objekt unter Verwendung einer optischen Vorrichtung mit einer numerischen Apertur;
Erhalten von Speckle-Daten auf Basis des durch die optische Vorrichtung optisch verarbeiteten Lichts; und
Steuern der numerischen Apertur der optischen Vorrichtung auf Basis der Speckle-Daten;
wobei das Objekt ein Fluid umfasst; wobei das Verfahren **dadurch gekennzeichnet ist, dass**
die numerische Apertur gesteuert wird, um eine Differenz zwischen Speckle-Daten des Fluids und Speckle-Daten eines Nicht-Fluids zu vergrößern.

**15.** Medizinisches Bildgebungssystem, umfassend eine Bildgebungsvorrichtung nach Anspruch 1.

**Revendications**

**1.** Appareil d'imagerie (1), comprenant :

une source de lumière (2) qui est configurée pour émettre de la lumière cohérente pour imager un objet comprenant un fluide ;
un appareil optique (3) qui est configuré pour traiter optiquement la lumière provenant de l'objet, l'appareil optique ayant une ouverture numérique ;
un imageur (4) qui est configuré pour recevoir la lumière traitée optiquement par l'appareil optique ; et
un circuit (5) configuré pour obtenir des données de tavelure basées sur la lumière reçue par l'imageur et contrôler l'ouverture numérique de l'appareil optique en fonction des données de tavelure ;
ledit appareil étant **caractérisé en ce que**
le circuit est configuré pour contrôler l'ouverture numérique de l'appareil optique pour augmenter une différence entre des données de tavelure du fluide et des données de tavelure d'un non-fluide.

**2.** Appareil d'imagerie de la revendication 1, dans lequel les données de tavelure sont un contraste de tavelure.

**3.** Appareil d'imagerie de la revendication 2, dans lequel le circuit est configuré pour contrôler l'ouverture numérique de l'appareil optique pour augmenter le contraste de tavelure.

**4.** Appareil d'imagerie de la revendication 3, dans lequel le circuit est configuré pour contrôler l'ouverture numérique de l'appareil optique pour maximiser le contraste de tavelure.

**5.** Appareil d'imagerie de la revendication 1, dans lequel le circuit est configuré pour produire des données-image basées sur les données de tavelure, les données-image indiquant un emplacement du fluide.

**6.** Appareil d'imagerie de la revendication 1, dans lequel les données de tavelure sont un contraste de tavelure et le circuit est configuré pour contrôler l'ouverture numérique de l'appareil optique pour maximiser une différence entre le contraste de tavelure du fluide et un contraste de tavelure d'un non-fluide.

**7.** Appareil d'imagerie de la revendication 1, dans lequel le fluide comprend du sang et l'appareil d'imagerie image du sang.

**8.** Appareil d'imagerie de la revendication 1, dans lequel le circuit comprend un contrôleur.

**9.** Appareil d'imagerie de la revendication 1, dans lequel l'imageur a une taille de pixel inférieure à une taille de grain de tavelure de la lumière provenant de l'objet.

**10.** Appareil d'imagerie de la revendication 1, la source de lumière étant une première source de lumière et l'appareil d'imagerie comprenant en outre :

une deuxième source de lumière qui émet de la lumière incohérente pour imager l'objet,
l'imageur imageant l'objet en utilisant la lumière incohérente.

11. Appareil d'imagerie de la revendication 1, dans lequel le circuit est configuré pour contrôler l'ouverture numérique de l'appareil optique en changeant une taille d'une ouverture dans l'appareil optique.

12. Appareil d'imagerie de la revendication 11, dans lequel l'appareil optique comprend un diaphragme.

13. Appareil d'imagerie de la revendication 11, dans lequel l'appareil optique comprend un modulateur optique spatial.

14. Procédé d'imagerie, comprenant :

l'émission de lumière cohérente pour imager un objet ;
le traitement optique de la lumière provenant de l'objet au moyen d'un appareil optique ayant une ouverture numérique ;
l'obtention de données de tavelure basées sur la lumière traitée optiquement par l'appareil optique ; et
le contrôle de l'ouverture numérique de l'appareil optique en fonction des données de tavelure ; dans lequel l'objet comprend un fluide ; ledit procédé étant **caractérisé en ce que**
l'ouverture numérique est contrôlée pour augmenter une différence entre des données de tavelure du fluide et des données de tavelure d'un non-fluide.

15. Système d'imagerie médicale, comprenant un appareil d'imagerie selon la revendication 1.

[Fig. 1]

[Fig. 2]

[Fig. 3]

[Fig. 4A]

[Fig. 4B]

[Fig. 5]

[Fig. 6]

[Fig. 7]

[Fig. 8]

[Fig. 9A]

[Fig. 9B]

[Fig. 9C]

[Fig. 10A]

[Fig. 10B]

[Fig. 10C]

[Fig. 10D]

[Fig. 11]

[Fig. 12]

[Fig. 13]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7113817 B1, WINCHESTER

- JP 2009136396 A

**Non-patent literature cited in the description**

- Speckle phenomena in optics: theory and applications. Roberts & Company, 7-23

- *Opt. Commun.,* 1981, vol. 37 (5), 325